# EUROPEAN PATENT SPECIFICATION

(11) **EP 2 655 620 B1**
(45) Date of publication and mention of the grant of the patent: **12.08.2015**
(21) Application number: 11801756.5
(22) Date of filing: 23.12.2011
(51) Int. Cl.: C12N 15/11, C12N 15/09, C12N 15/85

(54) **DNA EXPRESSION CONSTRUCT**
DNA-EXPRESSIONSKONSTRUKT
PRODUIT DE RECOMBINAISON D'ADN UTILISABLE À DES FINS D'EXPRESSION GÉNIQUE

(30) Priority: 23.12.2010 GB 201021873
(43) Date of publication of application: 30.10.2013
(73) Proprietor: Mologen AG, 14195 Berlin (DE)
(72) Inventor: SCHROFF, Matthias, 14195 Berlin (DE); KLEUSS, Christiane, 12203 Berlin (DE); KAPP, Kerstin, 10247 Berlin (DE)
(74) Representative: Tegethoff, Sebastian
(86) International application number: PCT/EP2011/073984
(87) International publication number: WO 2012/085282

(56) References cited:
- WO-A1-98/21322
- WO-A2-2004/016786
- WO-A2-2009/035554
- KIM YOUNGMI ET AL: "Superior structure stability and selectivity of hairpin nucleic acid probes with an L-DNA stem.", NUCLEIC ACIDS RESEARCH 2007 LNKD- PUBMED:17959649, vol. 35, no. 21, 2007, pages 7279-7287, XP007920248, ISSN: 1362-4962
- JUNJI KAWAKAMI ET AL: "Thermodynamic Analysis of Duplex Formation of the Heterochiral DNA with L-Deoxyadenosine", ANALYTICAL SCIENCES, vol. 21, no. 2, 1 February 2005 (2005-02-01), pages 77-82, XP55019354, ISSN: 0910-6340, DOI: 10.2116/analsci.21.77
- URATA H ET AL: "SYNTHESIS AND PROPERTIES OF MIRROR-IMAGE DNA", NUCLEIC ACIDS RESEARCH, OXFORD UNIVERSITY PRESS, SURREY, GB, vol. 20, no. 13, 1 January 1992 (1992-01-01), pages 3325-3332, XP002055870, ISSN: 0305-1048 cited in the application
- CHENXIANG LIN ET AL: "Mirror Image DNA Nanostructures for Chiral Supramolecular Assemblies", NANO LETTERS, vol. 9, no. 1, 14 January 2009 (2009-01-14), pages 433-436, XP55019361, ISSN: 1530-6984, DOI: 10.1021/nl803328v
- NICOLE C HAUSER ET AL: "Utilising the left-helical conformation of L-DNA for analysing different marker types on a single universal microarray platform", NUCLEIC ACIDS RESEARCH, OXFORD UNIVERSITY PRESS, GB, vol. 34, no. 18, 1 October 2006 (2006-10-01), pages 5101-5111, XP002667723, ISSN: 1362-4962, DOI: 10.1093/NAR/GKL671 cited in the application

## Description

### FIELD OF THE INVENTION

The invention relates to a minimalistic gene expression construct, its transfer into cells and its use for gene expression for molecular-medical applications.

### BACKGROUND OF THE INVENTION

Gene expression constructs that can be transferred into a cell of interest are frequently being used for DNA vaccination, tumour therapy and -prevention. Vector constructs for these purposes have to assure successful transfection and a maximum of safety for the patient. So-called plasmid-based constructs of a circular closed DNA double strand that are physically or chemically transfected into cells are relatively safe. However, depending on the cell type of interest, transfection efficiency is usually not satisfactory. Therefore, plasmids are not applicable for clinical protocols. In addition, they include usually several prokaryotic proteins (e.g., antibiotic resistance genes) that are being co-transferred into the cell. Prokaryotic promoters are not absolutely silent in eukaryotic cells which may result in unwanted immune effects. The host's immune system may also eliminate the transfected cells. Furthermore, unmethylated CG motifs in plasmid vectors can be immunostimulatory and account for at least some of the immunotoxicity observed in gene therapy protocols.

Replication-defective retroviral or adenoviral vectors are often being employed in clinical protocols due to their much higher transfection efficiency compared to plasmids. However, they harbour the substantial risk of recombination with wild-type viruses or activation of oncogenes. In addition, the necessity to use high viral titres may cause inflammation.

Therefore, the development of improved gene expression constructs is essential to overcome the unwanted side effects of plasmids and viral vectors. The EP 0 941 318 discloses a small, linear, covalently closed, dumbbell-shaped new vector type for minimalistic, immunologically defined gene expression (MIDGE). MIDGE is a minimal-size gene transfer unit; it only contains the minimum of sequences necessary: the expression cassette, including promoter, gene, and RNA-stabilizing sequence, flanked by two short hairpin oligonucleotide sequences. In addition, the design of MIDGE provides means for the effective transport into a cell of interest.

MIDGE molecules are much smaller than the conventionally used plasmids or viral vectors and are therefore easier to transfer into cells and allow high expression rates. They include short hairpins at both ends to protect the expression cassette against degradation by nucleases.

### BRIEF SUMMARY OF THE INVENTION

With regard to the state of the art it is an objective of the present invention to provide an effective DNA construct for gene expression in a eukaryotic cell, which is protected against degradation by nucleases.

According to the disclosure a DNA construct for gene expression is provided, wherein the construct is a linear and open-chained DNA double strand comprising a promoter sequence, a coding sequence and a termination signal, wherein the construct comprises at least one L-DNA nucleotide within the last five nucleotides of a 5'- and/or a 3'-end. A linear partly double-stranded dumbbell-shaped DNA construct comprising at least one L-DNA nucleotide is also within the scope of the present disclosure.

A DNA construct is further provided, wherein at least one L-DNA nucleotide is located at the 5'- and/or the 3'-end. A single stranded loop can be located at the 5'- and/or the 3'-end, also in combination with at least one L-DNA nucleotide, which is located within the last 5 nucleotides of the 5'- and/or the 3'-end.

A construct according to the disclosure may comprise a partially or completely double-stranded DNA chain. It is additionally intended that at least one L- or D-DNA nucleotide is modified with a functional group selected from the group comprising carboxyl, amine, amide, aldimine, ketal, acetal, ester, ether, disulfide, thiol and aldehyde groups.

The nucleotide can be linked to a compound selected from the group comprising peptides, proteins, carbohydrates, lipids, vesicles, antibodies, synthetic molecules, polymers, micro projectiles, metal particles, nanoparticles, or a solid phase.

It is intended that a construct according to the present disclosure comprises a promoter, which is selected from the group comprising promoter sequences, which are operable in a human beings, animals or eukaryotic cells. The construct may encode proteins, peptides, antibodies, hormones, cytokines or other biologically active substances, such as inhibitory, regulatory, or stimulatory RNAs or immunomodulators.

A further object of the present disclosure is the above-described DNA construct for stable or transient transfection of human, animal or eukaryotic cells as well as for gene therapy or DNA vaccination. The term "gene therapy" includes *in vivo* or *ex vivo,* as well as autologous or allogeneic approaches.

A further object of the present disclosure is the above-described DNA construct for prophylactic or therapeutic vaccination against infectious diseases or parasites.

A further object is the DNA construct according to the present disclosure for the treatment of cancer or autoimmune diseases, optionally in conjunction with the use of a non-coding immunomodulatory DNA construct.

It is a further object of the present invention to provide a pharmaceutical composition comprising a DNA construct as disclosed above. Such a pharmaceutical may further comprise a chemotherapeutic. Alternatively, it may contain an additional antigen of bacterial, fungal, parasitic, or viral origin.

### DETAILED DESCRIPTION OF THE INVENTION

Within the meaning of the present disclosure a linear open-chained DNA sequence is designated as DNA construct. Said DNA sequence can be single-stranded or partially or completely double-stranded. The term *DNA construct, DNA molecule,* and *expression construct* are used synonymously and do not indicate a limitation of the length of the corresponding DNA sequence. The monomeric components of DNA constructs are nucleotides.

A DNA construct can be manufactured synthetically or be partially or completely of biological origin, wherein a biological origin includes genetically based methods of manufacture of DNA sequences.

L-DNA or nucleotides in L-conformation refer to nucleotides which comprise L-deoxyribose as the sugar residue instead of the naturally occurring D-deoxyribose. L-deoxyribose is the enantiomer (mirror-image) of D-deoxyribose. DNA constructs partially or completely consisting of nucleotides in L-conformation can be partially or completely single-or double-stranded; however, nucleotides in L-conformation cannot hybridize to nucleotides in D-conformation (Hauser et al., Nucleic Acid Res. 2006 34: 5101-11). L-DNA is equally soluble and selective as D-DNA. Yet, L-DNA is resistant towards enzymatic degradation by naturally occurring enzymes, especially exonucleases, so L-DNA is protected against biological degradation (Urata et al., Nucleic Acids Res. 1992 20: 3325-32). Therefore, L-DNA is very widely applicable.

A "stem" according to the present disclosure shall be understood as a DNA double strand formed by base pairing either within the same DNA molecule (which is then partially self-complementary) or within different DNA molecules (which are partially or completely complementary). Intramolecular base-pairing designates base-pairing within the same DNA molecule and base-pairing between different DNA molecules is termed as intermolecular base-pairing.

A "loop" within the meaning of the present disclosure shall be understood as an unpaired, single-stranded region either within or at the end of a stem structure. A "hairpin" is a distinct combination of a stem and a loop, which occurs when two self-complementary regions of the same oligonucleotide hybridize to form a stem with an unpaired loop at one end.

A dumbbell-shape describes a linear DNA construct with hairpins at both ends flanking a stem region. Thus, a "linear DNA construct" within the context of the present disclosure describes either a linear open-chained DNA construct comprising single or double-stranded DNA or a linear dumbbell-shaped DNA construct comprising single stranded loops at both ends of a double stranded DNA stem.

The term "DNA end" whether meaning a 5'- or 3'end of a DNA single strand refers not only to the terminal nucleotide, but comprises the terminal three nucleotides or even the last five nucleotides with regard to the respective DNA end. A modification of a DNA end relates to at least one of the respective nucleotides.

A "solid phase" to which the nucleotides are covalently or non-covalently attached refers to, but is not restricted to, a column, a matrix, beads, glass including modified or functionalized glass, silica or silica-based materials including silicon and modified silicon, plastics (comprising polypropylene, polyethylene, polystyrene and copolymers of styrene and other materials, acrylics, polybutylene, polyurethanes etc.), nylon or nitrocellulose, resins, polysaccharides, carbon as well as inorganic glasses and plastics. Thus, microtiter plates are also within the scope of a solid phase according to the present disclosure.

Immunomodulation according to the present disclosure refers to immunostimulation and immunosuppression. Immunostimulation means preferentially that effector cells of the immune system are stimulated in order to proliferate, migrate, differentiate or become active in any other form. B cell proliferation for instance can be induced without co-stimulatory signals by immunostimulatory DNA molecules, which normally require a co-stimulatory signal from helper T cells.

Immunosuppression on the other hand shall be understood as reducing the activation or efficacy of the immune system. Immunosuppression is generally deliberately induced to prevent for instance the rejection of a transplanted organ, to treat graft-versus-host disease after a bone marrow transplant, or for the treatment of autoimmune diseases such as, for example, rheumatoid arthritis or Crohn's disease.

In this context, immunomodulation may also refer to the influence of the nature or the character of an immune reaction, either by affecting an immune reaction which is still developing or maturing or by modulating the character of an established immune reaction.

The term "vaccination" used in this disclosure refers to the administration of antigenic material (a vaccine) to produce immunity to a disease. Vaccines can prevent or ameliorate the effects of infection by many pathogens such as viruses, fungi, protozoan parasites, bacteria but also of allergic diseases and asthma, as well as of tumors. Vaccines typically contain one or more adjuvants, e.g. immunostimulatory nucleic acids, used to boost the immune response. Vaccination is generally considered to be the most effective and cost-effective method of preventing infectious and other diseases.

The material administered can, for example, be living but weakened forms of pathogens (such as fungi, bacteria or viruses), killed or inactivated forms of these pathogens, purified material such as proteins, nucleic acids encoding antigens, or cells such as tumor cells or dendritic cells. In particular, DNA vaccination has recently been developed. DNA vaccination works by insertion (and expression, triggering immune system recognition) of DNA encoding antigens into human or animal cells. Some cells of the immune system that recognize the proteins expressed will mount an attack against these proteins and against cells expressing them. One advantage of DNA vaccines is that they are very easy to produce and store. In addition, DNA vaccines have a number of advantages over conventional vaccines, including the ability to induce a wider range of immune response types.

Vaccination can be used as a prophylactic approach, leading to immunity against the antigen in the vaccinated, healthy individual upon exposure to the antigen. Alternatively, a therapeutic vaccination can cause an improved response of the immune system of the vaccinated, diseased individual, by guiding the immune system of the individual towards the antigens. Both prophylactic and therapeutic vaccination can be applied to humans as well as animals.

The term "gene therapy" used in this disclosure refers to the transient or permanent genetic modification (e.g. insertion, alteration, or removal of genes) of an individual's cells and/or biological tissues in order to treat diseases, such as tumors or autoimmune diseases. The most common form of gene therapy involves the insertion of functional genes into an unspecified genomic location in order to replace a mutated gene, but other forms involve directly correcting the mutation or modifying a normal gene that enables a viral infection or even transferring a gene or a gene fragment into a cell for its transcription.

"Autologous gene therapy" refers to using tissues or cells of the selfsame individual. The isolated cells or tissues will be modified by gene therapy and reintroduced into the donor. In contrast, "allogenic gene therapy" refers to using cells for gene therapy from an individual other than the acceptor individual. After genetic modification, the allogenic cells are introduced into the acceptor.

The term *"ex-vivo* gene therapy" refers to a therapy approach in which cells from an individual, e.g. hematopoietic stem cells or hematopoietic progenitor cells, are genetically modified *ex vivo* and subsequently introduced to the individual to be treated. The term "*in-vivo* gene therapy" refers to a therapy approach in which cells from an individual, e.g. hematopoietic stem cells or hematopoietic progenitor cells, are genetically modified *in vivo,* using viral vectors or other expression constructs for example.

Gene therapy may also be classified into "germ line gene therapy" and "somatic gene therapy". In case of "germ line gene therapy", germ cells, i.e., sperm or ova, are genetically modified. The genetic changes are ordinarily integrated into their genomes. Therefore, the change due to therapy would be heritable and would be passed on to later generations. This approach is useful for treatment of genetic disorders and hereditary diseases. In case of "somatic gene therapy", the therapeutic genes are transferred into the somatic cells of an individual. Any modifications and effects will be restricted to the individual only, and will not be inherited by the individual's offspring or later generations.

The term "cancer" comprises cancerous diseases or a tumor being treated or prevented that is selected from the group comprising mammary carcinomas, melanoma, skin neoplasms, gastrointestinal tumors, including colon carcinomas, stomach carcinomas, pancreas carcinomas, colon cancer, small intestine cancer, ovarial carcinomas, cervical carcinomas, lung cancer, prostate cancer, kidney cell carcinomas and/or liver metastases.

Autoimmune diseases according to the present disclosure comprise rheumatoid arthritis, Crohn's disease, systemic lupus (SLE), autoimmune thyroiditis, Hashimoto's thyroiditis, multiple sclerosis, Graves' disease, myasthenia gravis, celiac disease and Addison's disease.

Infectious diseases according to the present invention comprise infections by bacteria, viruses, fungi, or eukaryotic parasites, such as HIV, Hepatitis, flu, leishmaniosis, bacterial pneumonia, tuberculosis, measles, pertussis, tetanus, meningitis, syphilis, malaria and cholera, as well as animal-specific diseases such as Feline Immunodeficience Virus infections (FIV), Feline Leukemia Virus infections (FeLV), Bovine Herpes Virus infections (BHV), and Bovine viral Diarrhea.

A L-DNA-comprising desoxyribonucleic acid construct comprising a minimal gene expression unit results in a gene expression construct, which will not be degraded by nucleases. Experimental data demonstrate that such protected gene expression constructs are suitable for effective gene transfer and expression in a cell of interest.

The gene expression rates that can be achieved by transfection of constructs according to the present disclosure are higher than rates that can be achieved by the covalently closed molecules as disclosed in EP 0941318 (the MIDGE vector). The MIDGE vector has been shown to achieve higher transfection efficiencies as well as better expression rates than comparable plasmid vectors (Schakowski et.al., in vivo, 21:17-24,2007). By avoiding the single-stranded loops of EP 0941318 the molecule of the instant invention is further reduced in size which facilitates gene transfer. Due to the L-DNA modifications, stability is given. In fact, the use of DNA-degrading enzymes for the removal of unmodified DNA during the production process underscores this stability. It has to be noted that it is also within the scope of the present disclosure to provide a L-DNA comprising DNA construct with at least one hairpin arranged at one end of the double strand.

A DNA construct according to the present disclosure may be used for the artificial gene expression of the encoded gene in a cell of interest. In this regard, DNA vaccination, tumour therapy and -prevention require expression constructs which are safe and efficient to enable use in clinical protocols. Standard expression constructs are, for example, plasmid-based vectors. However, these vectors have two disadvantages. First, their efficiency is rather low and second, plasmid-based vectors comprise several genes and genetic elements unnecessary for the expression of the desired polypeptide and thereby carry the danger of unpredictable immunological side effects. By longer and repeated application it is likely that the desired immune response is masked by these side effects due to severe complications that may arise.

Other expression constructs of the state of the art include replication-defective retroviral or adenoviral vectors. Although they are very efficient in that they can be employed for transducing even non-dividing cells of a wide variety of cell types, they harbour the risk of recombining with wild-type viruses and thereby creating new pathogenic viruses as well as activation of oncogenes. In addition, viral proteins are very likely to cause immunological side effects, especially at high titres.

The minimal expression units of expression constructs according to the present disclosure may encode but are not limited to MHC-I or MHC-II presentable peptides, cytokines, or components of the regulation of the cell cycle, or regulatory RNA molecules and antisense RNA, ribozymes or mRNA-editing RNA molecules. In addition, the DNA constructs according to the disclosure allow their adsorption or covalent or ionic binding to e.g. peptides, proteins, carbohydrates, lipids, or glycopeptide ligands, as well as to micro projectiles which allow transferring the constructs into cells by ballistic transfer, especially into dermis, muscle tissue, pancreas, and the liver.

### BRIEF SUMMARY OF THE FIGURES

The disclosure will be further illustrated by examples and figures without being limited to the disclosed embodiments. It shows:
- Fig. 1: Schematic illustration of a DNA construct according to the disclosure
- Fig. 2: Agarose gel electrophoresis of DNA constructs after enzymatic digestion
- Fig. 3: Transfection efficiency using electroporation of L-DNA construct 2L-M
- Fig. 4: Transfection efficiency using lipofection of L-DNA construct 2L-M
- Fig. 5: Fluorescence intensity comparing L- and D-DNA expression constructs
- Fig. 6: Comparison of transfection efficiency using electroporation of L-DNA constructs DL-M and LD-M and the MIDGE vector
- Fig. 7: Immunisation of mice with MIDGE or L-MIDGE encoding small protein of Hepatitis B surface antigen

### DETAILED DESCRIPTION OF THE FIGURES

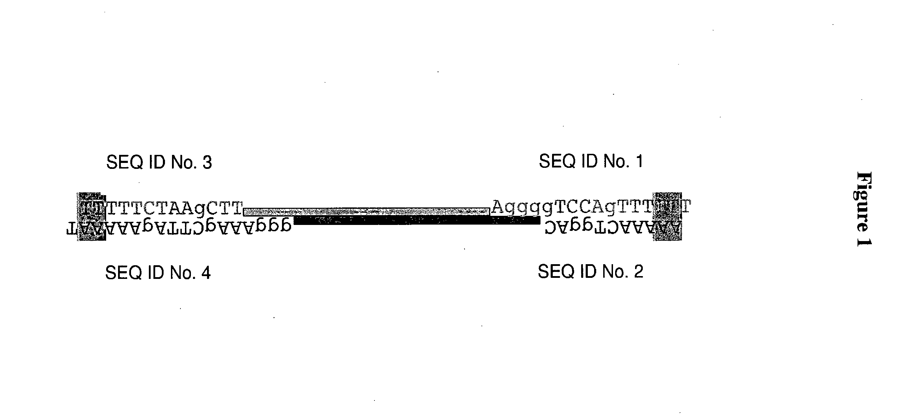

Figure 1 shows a schematic illustration of a DNA construct (2L-M) according to the present disclosure. As indicated in Table 1 the terminal oligonucleotides comprise nucleotides in L-conformation, which are indicated by the grey boxes in figure 1 at the end of the double stranded construct. Thus, the whole DNA construct is protected against degradation by exonucleases. The construct depicted in figure 1 does not have or need a hairpin loop at one or both ends in order to be protected against degradation.

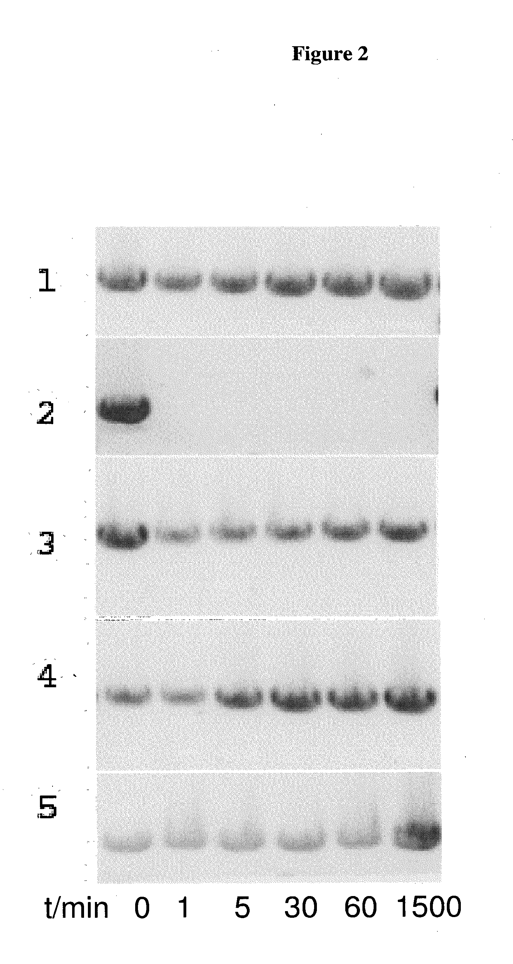

Figure 2 shows an agarose gel of DNA constructs being subjected to digestion by the T7-Polymerase from the T7 bacteriophage. 6 µg of each DNA construct were incubated with 10 units of T7-Polymerase, with a total reaction volume of 20 µl. After 0, 1, 5, 30, 60, and 1500 minutes, an aliquot of 3µl of incubation mixtures was removed from the sample and diluted with 5µl of formamide-containing Sanger dye. All aliquots were loaded onto a 3% agarose gel, which was run at 100 Volt for 100 minutes.

Lane 1 shows the MIDGE vector according to EP 0 941 318. Lane 2 shows the unprotected expression cassette used for the manufacture of the MIDGE vector according to EP 0 941 318. Lane 3 shows the DNA construct according to the present invention, with both ends protected by L-DNA (referred to subsequently as "2L-M"). Lanes 4 and 5 show the alternative DNA constructs according to the present invention, with one end protected by L-DNA, and the other by a hairpin. The DNA construct in Lane 4 contains the L-DNA-protected end on the 5' end of the promoter and a hairpin on the 3' end, while the construct of Lane 5 is protected by L-DNA on the 3' end from the promoter, and a hairpin on its 5' end, respectively. These constructs are referred to as "DL-M" and "LD-M" (see Examples section for details).

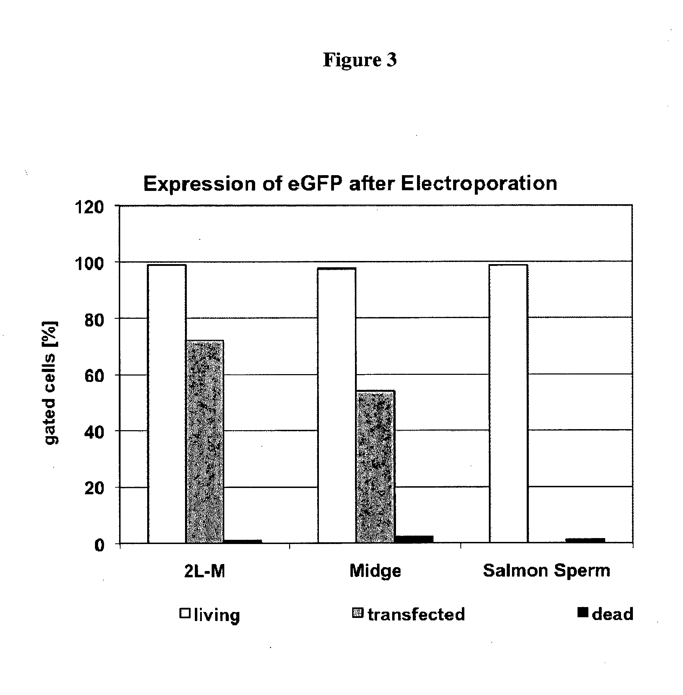

Figure 3 shows the transfection efficacy using electroporation of the 2L-M L-DNA comprising expression construct according to the present disclosure (2L-M) in comparison to a MIDGE as disclosed in EP 0 941 318 (M), both encoding eGFP. The experimental results using salmon sperm as negative control are shown on the right side. The left black bar represents the number of living cells, the grey middle bar indicates the number of transfected cells and the white right bar shows the number of dead cells. The black line indicates the total number of cells after electroporation.

The transfection efficiency of the new 2L-M L-DNA comprising construct is about one third higher than using the dumbbell-shaped MIDGE construct. As mentioned above, the MIDGE vector has been shown to possess a higher transfection efficacy than comparable plasmid constructs (Schakowski et.al., in vivo, 21:17-24,2007).

The results do not only demonstrate that the expression construct according the present disclosure is suitable for gene expression, but also show surprisingly that the construct has an unexpected higher efficiency in comparison to other expression constructs. The disclosed expression construct is obviously stable enough to cause a considerable amount of gene expression and on the other hand the uptake or transfer of the construct into cells seems to work quite well, both when using electroporation, as well as using lipofection.

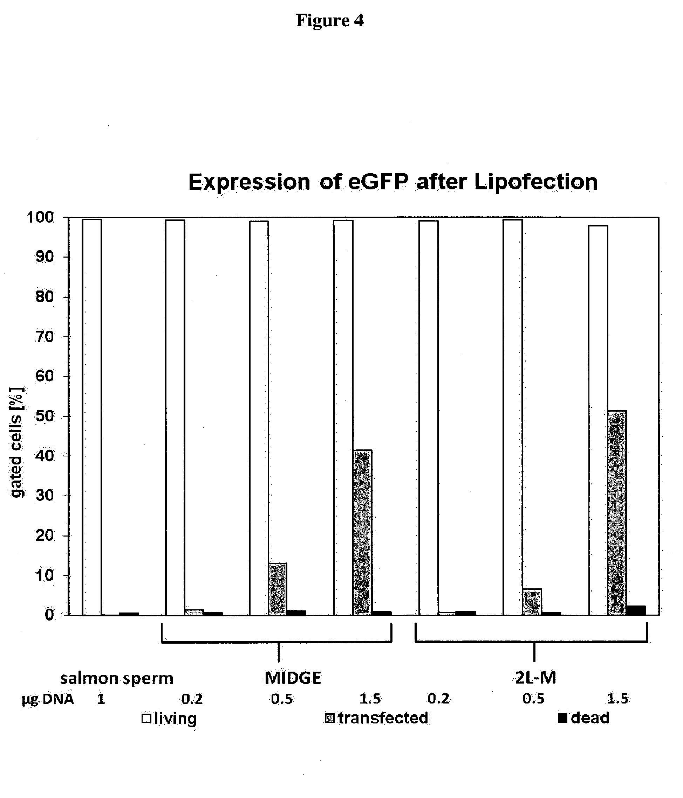

In order to check whether the results shown in Figure 3 are related to electroporation, equimolar amounts of MIDGE as disclosed in EP 0 941 318 and L-DNA comprising DNA constructs 2L-M were transferred into cells by lipofection (Figure 4). Salmon sperm was used as negative control. Each white bar shows the number of living cells, the grey bar the number of transfected cells, and the black bar indicates the number of dead cells.

Using 1.5 µg DNA for lipofection results in a higher number of transfected cells for the 2L-M L-DNA construct according to the present disclosure. Although the number of transfected cells using 0.5 µg DNA is lower when using the L-DNA construct, the intensity of fluorescence was higher with the L-DNA construct.

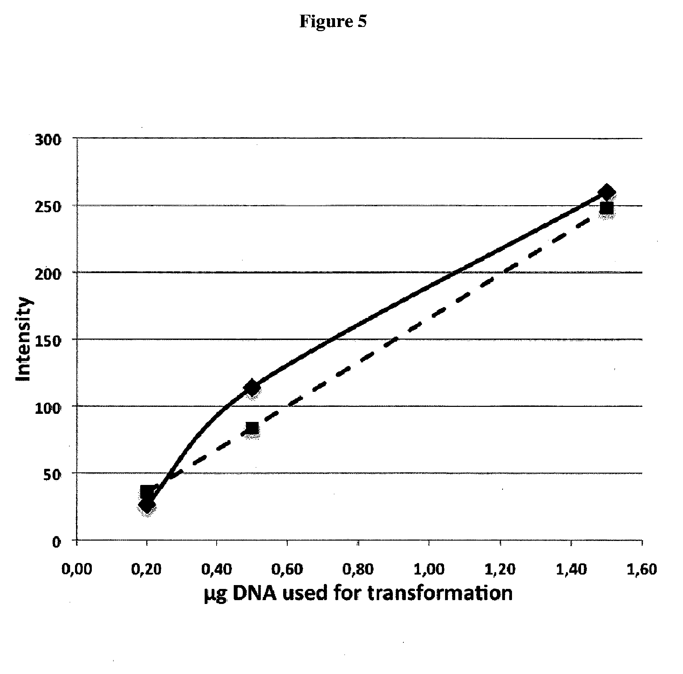

Figure 5 shows the relation between amount (mass) of DNA used for transfection and the mean fluorescence intensity evoked in individual cells. The solid line represents a 2L-M construct according to the invention and the dotted line shows the results using a MIDGE as disclosed in EP 0 941 318.

In order to receive an equal light intensity about 30% more DNA (mass) of a dumbbell-shaped DNA construct has to be applied, compared to the 2L-M construct disclosed. Consequently, the efficiency of the DNA construct according to the present disclosure is surprisingly higher than using a dumbbell-shaped construct.

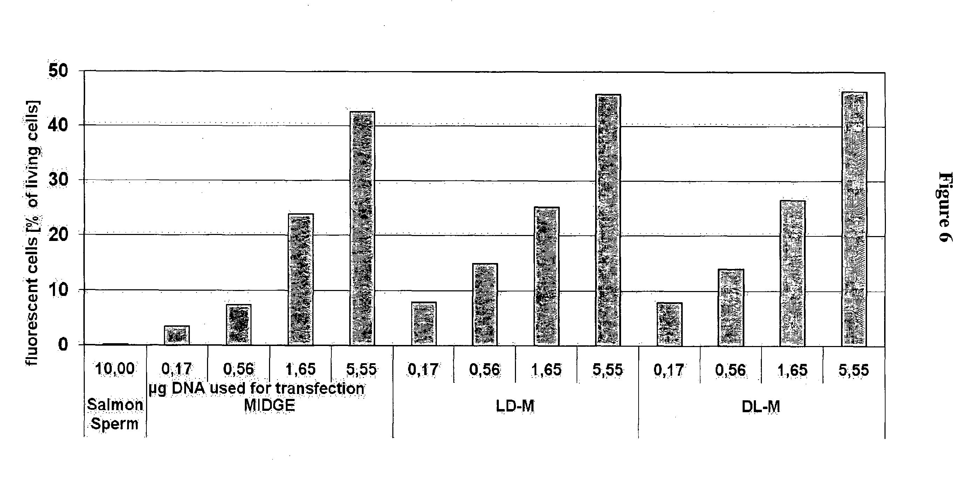

Figure 6 compares the fluorescence obtained from CHO-K1 cells which were modified with eGFP via electroporation using the MIDGE vector and the constructs LD-M, and DL-M, as well as salmon sperm as control. Surprisingly, at all concentrations used, the DL-M and LD-M constructs both caused a higher fluorescence signal from the cells, as compared to the MIDGE vector. Thus, the L-DNA constructs containing one L-DNA-modified end and one D-DNA hairpin surprisingly showed an increased transfection efficiency as compared to the MIDGE vector. Clearly, these constructs are taken up quite well by the cells and allow for a stable and efficient expression, resulting in an improved performance by the vectors.

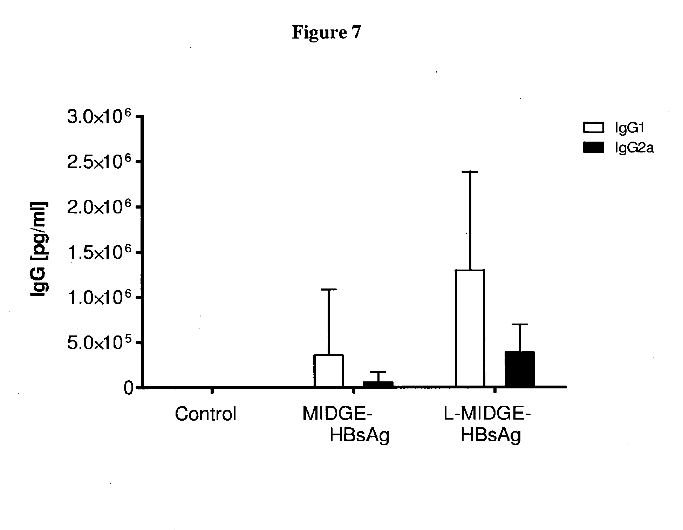

To test the efficacy of the disclosed expression constructs in an animal model, a DNA sequence coding for the small protein of Hepatitis B surface antigen (HBsAg) was used. A linear representation showing significant differences between groups of mice immunised with MIDGE and L-MIDGE (in case of IgG2a, p=0.033) is depicted in Figure 7. Using L-MIDGE as expression construct resulted in higher IgG antibody concentrations than the use of MIDGE.

The present disclosure provides a linear construct for gene expression, which is protected against degradation by nucleases and suitable for efficient gene expression after transfection into cells. It was possible to demonstrate that the gene products resulting from the transfection of expression constructs according to the disclosure are inducing specific antibodies. Thus, it has been proven that the disclosed expression constructs are suitable for the efficient gene expression in eukaryotic cells.

### EXAMPLES

### Manufacture of DNA constructs

The manufacture of the DNA constructs according to the disclosure resembles that of EP 0 941 318. However, the hairpin oligonucleotides are replaced by so-called "L-adapters", as summarized in Table 1.Both adapters were composed of the individual chimeric DNA molecules SEQ ID No. 1 and SEQ ID No. 2 (L-adapter 1) or SEQ ID No. 3 and SEQ ID No. 4 (L-adapter 2), respectively (Table 1). The adapters were generated by hybridization of equimolar concentrations of the single-stranded DNA molecules according to table 1 at 0.28 mg/ml for 40 min at gradually decreasing temperatures from 90°C to 25°C in 40 mM Tris-HCl, 10 mM MgCl2, 10 mM DTT, 0.5 mM ATP (pH 7.8 at 25°C). After ligation of both adapters to the expression cassette as synthesized in EP 0 941 318 the subsequent removal of linear D-DNA by T7 polymerase and the final HPLC purification of the product was conducted. This construct is referred to as "2L-M".

Alternatively, one of the L-adapters was replaced by the corresponding hairpin, as used in the MIDGE vector described in EP 0 941 318. This resulted in DNA constructs with an L-DNA protection at one side of the promoter, and a hairpin on the other side. These constructs are referred to as "DL-M" (L-adapter 1 and hairpin) and "LD-M" (L-adapter 2 and hairpin), respectively.

**Table 1: DNA molecules used for production of the DNA constructs. All nucleotides are in D-conformation except for the designated nucleotides.**

| **SEQ ID** | **Name** | **Sequence (5'-3')** | **Nucleotides in L-conformation** |
|---|---|---|---|
| SEQ ID No. 1 | CKm364 | AGGGGTCCAGTTTTTT | 14, 15 |
| SEQ ID No. 2 | CKm365 | AAAAACTGGAC | 1, 2 |
| SEQ ID No. 3 | CKm362 | TTTTTCTAAGCTT | 1, 2 |
| SEQ ID No. 4 | CKm363 | GGGAAAGCTTAGAAAAAT | 16, 17 |
| | L-adapter 1 | SEQ ID No. 1 / SEQ ID No. 2 | see above |
| | L-adapter 2 | SEQ ID No. 3 / SEQ ID No. 4 | see above |

### Transfection

In order to transfer the DNA constructs into the cells, different transfection methods can be employed, for example lipofection or electroporation. Lipofection was carried out as follows: 6 x 10⁴ CHO-K1 cells were seeded on 3.8 cm² tissue culture matrix and transfected 24 h later with the indicated amounts of eGFP-expressing DNA by mixing 1:4 with Fugene HD (Roche) and processed as advised by the manufacturer. One day after transfection, cells were harvested by trypsinization and analyzed for fluorescence by flow cytometry (10.000 events counted).

### Electroporation

Electroporation was carried out as follows: 2 x 10⁶ CHO-K1 cells were resuspended in 500 µl growth medium, mixed with the indicated amounts of eGFP-expressing DNA plus 11 µg salmon sperm and pulsed with 270 V at 1650 µF. Subsequently, cells were seeded on 9.5 cm² tissue culture matrix and cultivated. One day after transfection, cells were harvested by trypsinization and analyzed for fluorescence by flow cytometry (10,000 events counted).

### Immunisation of Mice

The HBsAg coding sequence was placed under control of the strong viral P_{CMV} promoter. L-MIDGE vectors encoding HBsAg were produced using the ODNs CKm362-365 (comp. table 1), MIDGE vectors encoding HBsAg were produced according to EP 0 941 318. Balb/c mice (6 animals per group) were immunised intradermally with 10 µg/25 µl L-MIDGE-HBsAg vectors (8.14 pmol) or 10 µg/25 µl MIDGE-HBsAg vectors (8.179 pmol) twice 3 weeks apart. Two weeks after the second immunisation, serum was obtained and analysed via ELISA for HBsAg-specific IgG1 and IgG2a antibodies using HBsAg-coated ELISA plates (Dade Behring; Enzygnost Anti-HBs II; cat. no. OQNE17 or OQNE11), Rat-anti-mouse IgG1 and IgG2a (BD; cat. nos. 559626 and 553391) as secondary antibodies, Mouse-anti-HBsAg IgG2a (Affinity BioReagents, cat. no. MA1-19264) as standard and Mouse-anti-HBsAg IgG1 (Affinity BioReagents, cat. no. MA1-19263) as standard.

## Claims

1. A DNA construct for gene expression, wherein the construct is a linear and open-chained DNA double strand comprising a promoter sequence, a coding sequence and a termination signal, wherein the construct comprises at least one L-DNA nucleotide and wherein at least one L-DNA nucleotide is comprised within the last five nucleotides of a 5'- and/or a 3'-end.

2. The construct according to claim 1, wherein at least one end of the DNA construct comprises a single stranded loop.

3. The construct according to any of the preceding claims, wherein said construct is partially or completely double-stranded.

4. The construct according to any of the preceding claims, wherein at least one L- or D-DNA nucleotide is modified with a functional group selected from the group comprising carboxyl, amine, amide, aldimine, ketal, acetal, ester, ether, disulfide, thiol and aldehyde groups.

5. The construct according to claim 4, wherein a modified nucleotide is linked to a compound selected from the group comprising peptides, proteins, carbohydrates, antibodies, synthetic molecules, polymers, micro projectiles, metal particles, nanoparticles, lipids, or a solid phase.

6. The construct according to any of the preceding claims, wherein the promoter is selected from the group comprising promoter sequences, which are operable in a human being, animals or eukaryotic cells.

7. The construct according to at least one of the preceding claims, wherein said construct encodes proteins, peptides, antibodies, hormones, cytokines or other biologically active substances.

8. The construct according to claim 7, wherein biologically active substances are immunomodulators.

9. A pharmaceutical composition comprising the DNA construct according to any of the claims 1 to 8.

10. The pharmaceutical composition according to claim 9 comprising additionally a chemotherapeutic.

11. The DNA construct according to any one of claims 1 to 8 or the pharmaceutical composition according to claims 9 or 10 for stable or transient in vitro transfection of a human, animal or eukaryotic cell.

12. The DNA construct according to any one of claims 1 to 8 or the pharmaceutical composition according to claims 9 or 10 for *ex-vivo* gene therapy or DNA vaccination.

13. The DNA construct according to any one of claims 1 to 8 or the pharmaceutical composition according to claims 9 or 10 for use in a method for the treatment of cancer or autoimmune diseases.

14. A combination of the DNA construct according to any one of claims 1 to 8 or the pharmaceutical composition according to claims 9 or 10 with a non-coding immunomodulatory DNA construct.

## Patentansprüche

1. Ein DNA-Konstrukt zur Genexpression, wobei das Konstrukt ein linear und offenkettiger DNA-Doppelstrang ist, umfassend eine Promotersequenz, eine kodierende Sequenz und ein Terminationssignal, wobei das Konstrukt wenigstens ein L-DNA-Nukleotid umfasst und wobei wenigstens ein L-DNA-Nukleotid innerhalb der letzten fünf Nukleotide des 5' und/oder 3'-Endes umfasst ist.

2. Das Konstrukt nach Anspruch 1, wobei wenigstens ein Ende des DNA-Konstruktes einen einzelsträngigen Loop umfasst.

3. Das Konstrukt nach wenigstens einem der vorhergehenden Ansprüche, wobei das Konstrukt teilweise oder vollständig doppelsträngig ist.

4. Das Konstrukt nach einem der vorhergehenden Ansprüche, wobei wenigstens ein L- oder D-DNA-Nukleotid mit einer funktionalen Gruppe modifiziert ist ausgewählt aus der Gruppe umfassend Carboxyl, Armin, Armid, Aldimin, Ketal, Acetal, Ester, Ether, Disulfit, Thiol und Aldehyd Gruppe.

5. Das Konstrukt nach Anspruch 4, wobei ein modifiziertes Nukleotid an einen Stoff ausgewählt aus der Gruppe umfassend Peptide, Proteine, Carbohydrate, Antikörper, synthetische Moleküle, Polymere, Mikroprojektile, Metallpartikel, Nanopartikel, Lipide oder einer festen Phase gebunden ist.

6. Das Konstrukt nach wenigstens einem der vorhergehenden Ansprüche, wobei der Promoter ausgewählt ist aus der Gruppe umfassend Promotersequenzen, welche im Menschen, Tieren oder Eukaryoten-Zellen einsetzbar sind.

7. Das Konstrukt nach wenigstens einem der vorhergehenden Ansprüche, wobei besagtes Konstrukt für Proteine, Peptide, Antikörper, Hormone, Cytokine oder andere biologisch aktive Substanzen kodiert.

8. Das Konstrukt nach Anspruch 7, wobei biologisch aktive Substanzen Immunmodulatoren sind.

9. Eine pharmazeutische Zusammensetzung umfassend ein DNA-Konstrukt nach einem der Ansprüche 1 bis 8.

10. Die pharmazeutische Zusammensetzung nach Anspruch 9 umfassend zusätzlich ein Chemotherapeutikum.

11. Das DNA-Konstrukt nach einem der Ansprüche 1 bis 8 oder die pharmazeutische Zusammensetzung nach den Ansprüchen 9 oder 10 zur stabilen oder transienten In-Vitro-Transfektion von humanen, tierischen oder Eukarytonen-Zellen.

12. Das DNA-Konstrukt nach einem der Ansprüche 1 bis 8 oder die pharmazeutische Komposition nach Anspruch 9 oder 10 zur Ex-Vivo Gentherapie oder DNA-Vakzinierung.

13. Das DNA-Konstrukt nach einem der Ansprüche 1 bis 8 oder die pharmazeutische Komposition nach Anspruch 9 oder 10 zur Verwendung in einer Methode zur Behandlung von Krebs oder Autoimmunerkrankungen.

14. Eine Kombination des DNA-Konstruktes nach einem der Ansprüche 1 bis 8 oder die pharmazeutische Zusammensetzung nach den Ansprüchen 9 oder 10 mit einem nicht-kodierenden immunmodulatorischen DNA-Konstrukts.

## Revendications

1. Construction d'ADN pour l'expression génique, dans laquelle la construction est un double brin d'ADN linéaire et à chaîne ouverte comprenant une séquence de promoteur, une séquence de codage et un signal de terminaison, dans laquelle la construction comprend au moins un nucléotide L-ADN et dans laquelle le au moins un nucléotide L-ADN est compris à l'intérieur des derniers 5 nucléotides d'une extrémité 5' et/ou 3'.

2. Construction selon la revendication 1, dans laquelle au moins une extrémité de la construction d'ADN comprend une boucle simple brin.

3. Construction selon l'une quelconque des revendications précédentes, dans laquelle la construction est partiellement ou complétement à double brin.

4. Construction selon l'une quelconque des revendications précédentes, dans laquelle au moins un nucléotide L-ADN ou D-ADN est modifié avec un groupe fonctionnel choisi dans le groupe comprenant carboxyle, amine, amide, aldimine, cétal, acétal, ester, éther, disulfure, thiol et groupes aldéhyde.

5. Construction selon la revendication 4, dans laquelle un nucléotide modifié est couplé à un composé choisi dans le groupe constitué par les peptides, protéines, carbohydrates, anticorps, molécules synthétiques, polymères, micro projectiles, particules métal-liques, nanoparticules, lipides, ou une phase solide.

6. Construction selon l'une quelconque des revendications précédentes, dans laquelle le promoteur est choisi dans le groupe comprenant des séquences de promoteur qui peuvent être actionnées dans des cellules d'êtres humains, animales ou eucaryotes.

7. Construction selon au moins l'une des revendications précédentes, dans laquelle ladite construction code des protéines, peptides, anticorps, hormones, cytokines, ou d'autres substances biologiquement actives.

8. Construction selon la revendication 7, dans laquelle les substances biologiquement actives sont des immunomodulateurs.

9. Composition pharmaceutique comprenant la construction ADN selon l'une quelconque des revendications 1 à 8.

10. Composition pharmaceutique selon la revendication 9, comprenant en outre un chimiothérapique.

11. Construction ADN selon l'une quelconque des revendications 1 à 8 ou composition pharmaceutique selon la revendication 9 ou 10 pour transfection in vitro stable ou transitoire d'une cellule eucaryote, humaine, ou animale.

12. Construction ADN selon l'une quelconque des revendications 1 à 8 ou composition pharmaceutique selon les revendications 9 ou 10 pour thérapie génique ex vivo ou vaccination ADN.

13. Construction ADN selon l'une quelconque des revendications 1 à 8 ou composition pharmaceutique selon les revendications 9 ou 10 pour utilisation dans une méthode de traitement de cancer ou de maladies auto-immunes.

14. Combinaison de la construction d'ADN selon l'une quelconque des revendications 1 à 8 ou de la composition pharmaceutique selon la revendication 9 ou 10 avec une construction ADN immunomodulatrice non codante.


### SEQUENCE LISTING

<110> MOLOGEN AG
<120> DNA Expression Construct
<130> 80526GB
<160> 4
<170> PatentIn version 3.5
<210> 1
   <211> 16
   <212> DNA
   <213> Artificial Sequence
<220>
   <223> synthetic oligodeoxynucleotide
<220>
   <221> misc_feature
   <223> nucleotides 14 and 15 in L-conformation
<400> 1
   aggggtccag tttttt 16
<210> 2
   <211> 11
   <212> DNA
   <213> Artificial Sequence
<220>
   <223> synthetic oligodeoxynucleotide
<220>
   <221> misc_feature
   <223> nucleotides 1 and 2 in L-conformation
<400> 2
   aaaaactgga c 11
<210> 3
   <211> 13
   <212> DNA
   <213> Artificial Sequence
<220>
   <223> synthetic oligodeoxynucleotide
<220>
   <221> misc_feature
   <223> nucleotides 1 and 2 in L-conformation
<400> 3
   tttttctaag ctt 13
<210> 4
   <211> 18
   <212> DNA
   <213> Artificial Sequence
<220>
   <223> synthetic oligodeoxynucleotide
<220>
   <221> misc_feature
   <223> nucleotides 16 and 17 in L-conformation
<400> 4
   gggaaagctt agaaaaat 18